# EUROPEAN PATENT SPECIFICATION

(11) **EP 2 645 976 B1**
(45) Date of publication and mention of the grant of the patent: **02.08.2017**
(21) Application number: 11844232.6
(22) Date of filing: 30.11.2011
(51) Int. Cl.: A61F 13/49, A61F 13/53, A61F 13/533, A61F 13/536

(54) **DISPOSABLE DIAPER**
WEGWERFWINDEL
COUCHE JETABLE

(30) Priority: 30.11.2010 JP 2010267836
(43) Date of publication of application: 09.10.2013
(73) Proprietor: Unicharm Corporation, Ehime 799-0111 (JP)
(72) Inventor: YAMANAKA, Yasuhiro, Kanonji-shi Kagawa 769-1602 (JP); SHIMADA, Takaaki, Kanonji-shi Kagawa 769-1602 (JP); OHASHI, Naoto, Kanonji-shi Kagawa 769-1602 (JP)
(74) Representative: Dolleymores
(86) International application number: PCT/JP2011/006688
(87) International publication number: WO 2012/073499

(56) References cited:
- EP-A1- 2 189 562
- JP-A- 2003 033 397
- JP-A- 2003 180 744
- JP-A- 2004 298 454
- JP-A- 2007 117 111
- JP-A- 2007 117 111
- JP-A- 2007 130 818
- JP-A- 2008 264 076
- JP-A- 2009 131 417
- JP-U- H0 621 622
- US-A- 5 986 167

## Description

### {Technical Field}

The present invention relates to disposable diapers, more particularly to diapers for newborn babies.

### {Background}

Conventionally, disposable wearing articles are known having a bodily fluid absorbent structure formed with a high stiffness region. For example, JP 2005-152241 A (PTL 1) discloses a disposable wearing article including a liquid-pervious inner sheet, a liquid-impervious outer sheet and a bodily fluid absorbent structure interposed between these inner and outer sheets, wherein a given region of the bodily fluid absorbent structure is debossed to define a high stiffness region.

### {Citation List}

### {Patent Literature}

{PTL 1} JP 2005-152241 A

### {Summary}

### {Technical Problem}

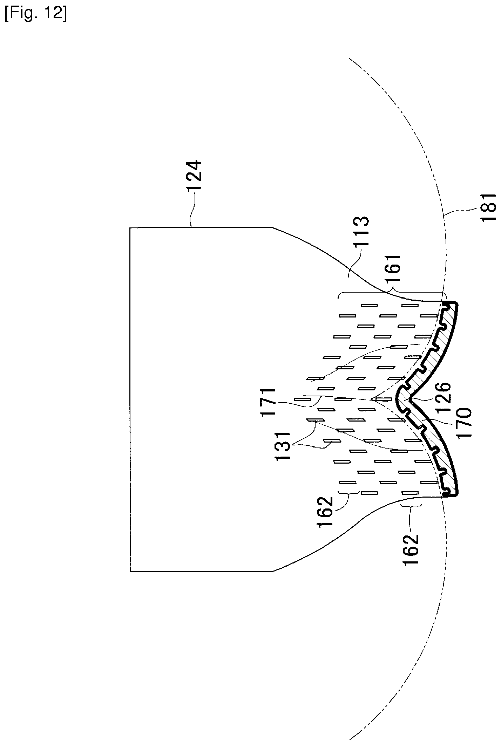

In the disposable wearing article disclosed in PTL 1, as will be apparent from Fig. 12 of the accompanying drawings, each of debosses 131 distributed in a high stiffness region 161 has a length dimension in a transverse dimension smaller than that in a longitudinal direction. Upon occurrence of urination, bodily fluids flow along these debosses 131 so that bodily fluids may be dispersed in the longitudinal direction and absorbed by an absorbent core 126.

In such a wearing article, however, a plurality of transverse high stiffness zones 162 are formed by arranging a plurality of longitudinally long debosses 131 in the transverse direction in a state of being substantially contiguous to one another in the longitudinal direction. In consequence, this wearing article includes no transverse low stiffness zone extending in the transverse direction and having none of the debosses 131.

When such a wearing article disclosed in PTL 1 is put on the wearer's body, a midsection of the crotch region 113 of the diaper is squeezed between the wearer's thighs and longitudinal creases 171 may appear in a bodily fluid absorbent structure 124 between adjacent debosses 131 or along extending directions of the debosses 131. As a consequence, the debosses 131 adjacent to one another in the longitudinal direction come into a substantially connected state in the proximity of the creases 171 in the longitudinal direction, and the bodily fluid absorbent structure 124 may therefore be formed with a ridge 170 along such connected lines, extending toward the wearer's bottom cleavage 181. Such a ridge 170 may dig into the wearer's bottom cleavage and not only make the wearer uncomfortable but also cause bodily fluids to move along the ridge and eventually leak sideways beyond the leg-openings. If for example the wearer is a newborn baby, the wearer often stays in a lying posture for a long time on a bed and, for example, when the wearer is picked up in a mother's arms, a relatively strong force is exerted on the bodily fluid absorbent structure 124 in the transverse direction. Consequently, while the shape of the ridges 170 is maintained, the longitudinal creases 171 in the bodily fluid absorbent structure 124 may be deepened.

### {Solution to Problem}

According to the present invention, there is provided the disposable diaper of independent claim 1. The dependent claims specify preferred but optional features.
A disposable diaper has a longitudinal direction and a transverse direction, including:
a chassis comprising a skin-facing side, a non-skin-facing side, a front waist region, a rear waist region and a crotch region extending in the longitudinal direction between the front and rear waist regions; and
a bodily fluid absorbent structure extending in the longitudinal direction across the crotch region into the front and rear waist regions wherein a region of the bodily fluid absorbent structure extending at least in a central area thereof is formed with a high stiffness region comprising a plurality of debosses spaced one from another in predetermined dimensions in the longitudinal and transverse directions.

In the disposable diaper, the bodily fluid absorbent structure includes an absorbent core and at least one sheet covering a surface of the absorbent core;
the debosses are shaped so that the length dimension of each deboss is longer in the transverse direction than in the longitudinal direction;
the high stiffness region includes a plurality of longitudinal low stiffness zones extending in the longitudinal direction and formed with none of the debosses, a plurality of transverse high stiffness zones formed with the debosses being arranged at regular intervals in the transverse direction, and transverse low stiffness zones lying between each pair of transverse high stiffness zones and being formed with none of the debosses;
the transverse low stiffness zones and transverse high stiffness zones are adjacent in the longitudinal direction;
in the high stiffness region, the width dimension of the transverse low stiffness zones in the longitudinal direction is larger than the width dimension of the longitudinal low stiffness zones in the transverse direction; and
the high stiffness region is formed in an area of the crotch region biased toward the front waist region.

### {Advantageous Effects of Invention}

In the disposable diaper according to the present invention, the debosses forming the high stiffness region of the bodily fluid absorbent structure are respectively shaped so that a length dimension thereof in the transverse direction (width) is longer than a length dimension thereof in the longitudinal direction (height) ; the high stiffness region includes the transverse high stiffness zones formed of the debosses intermittently arranged in the transverse direction; and the transverse low stiffness zones each defined between a pair of adjacent transverse high stiffness zones. With this unique arrangement, a plurality of the transverse creases can be easily developed on the bodily fluid absorbent structure of the diaper put on the wearer's body. Development of the transverse creases advantageously restricts development of the longitudinal creases in the longitudinal low stiffness zones which may cause the central portion of the bodily fluid absorbent structure to be convexly raised up toward the wearer's crotch region. In consequence, such ridge can be restricted as low as possible and such ridge should not dig into the wearer's bottom cleavage.

### {Brief Description of Drawings}

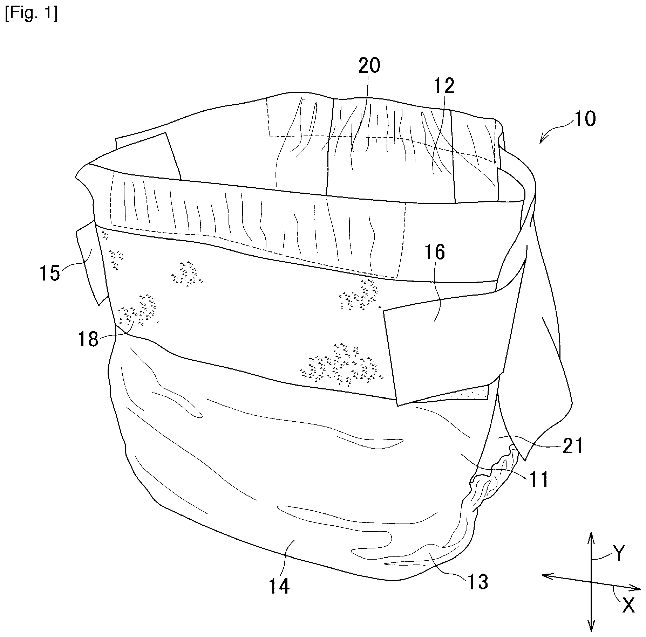

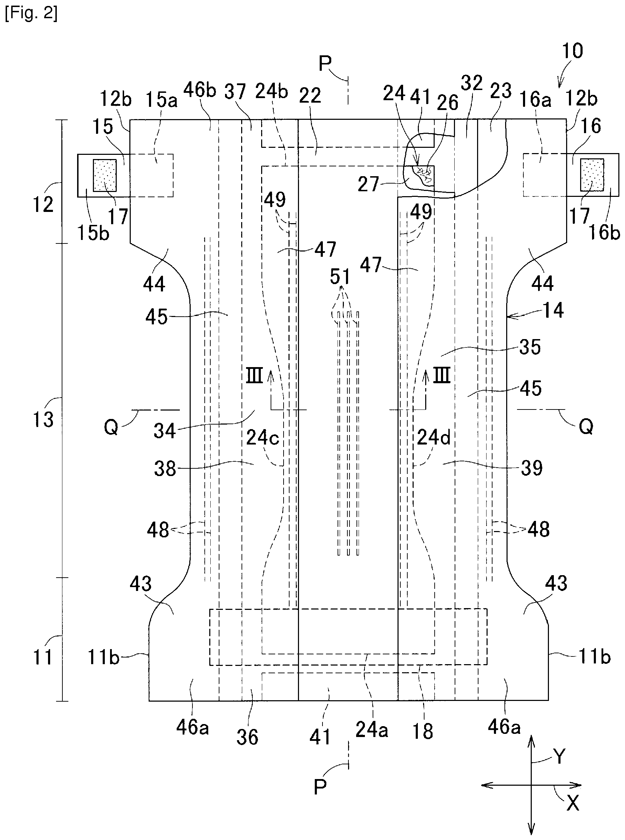

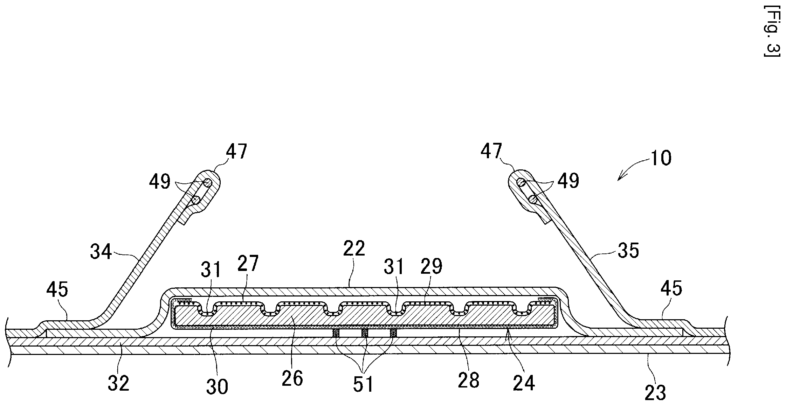

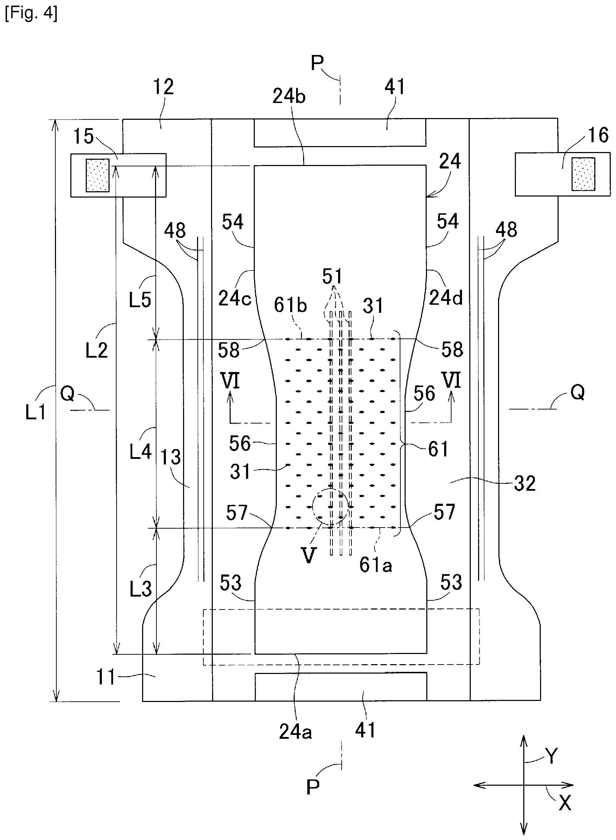

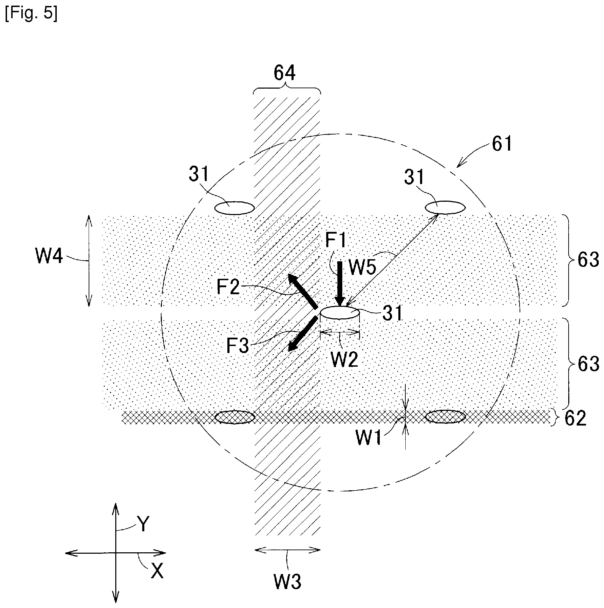

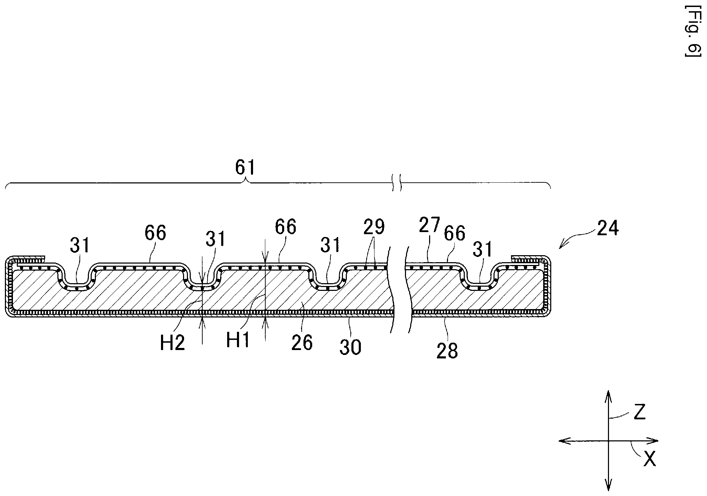

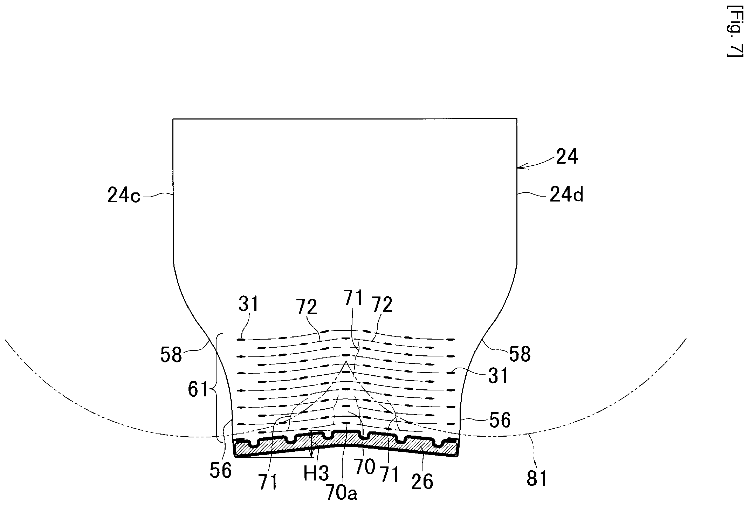

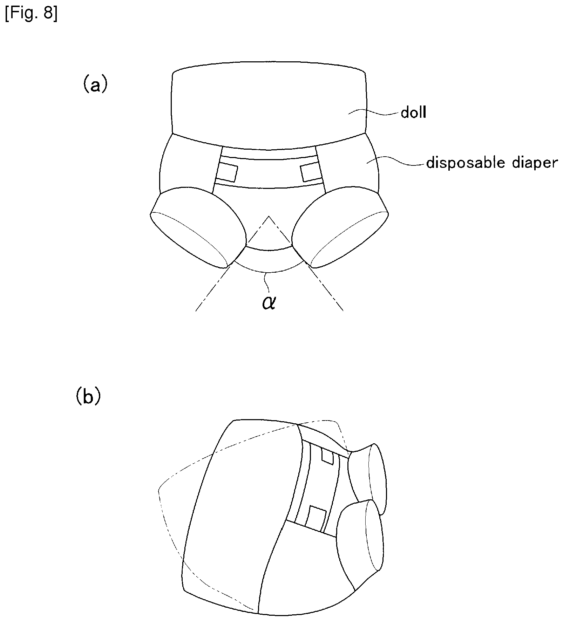

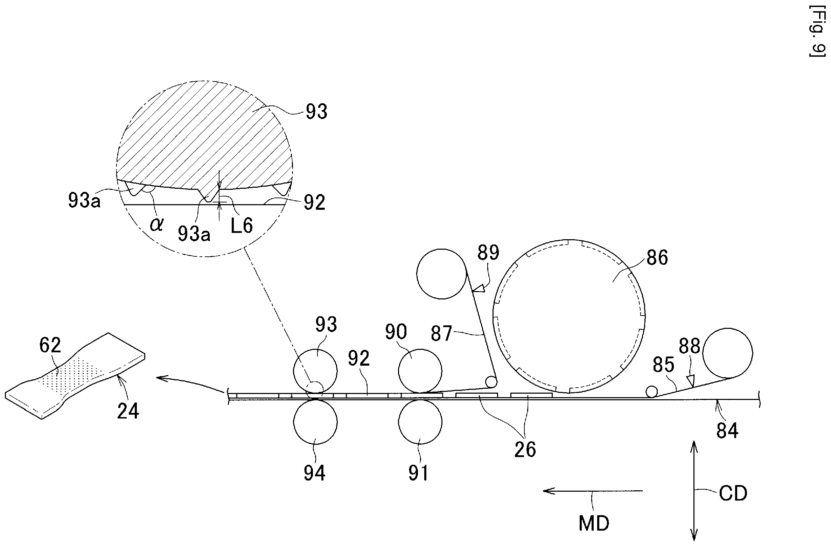

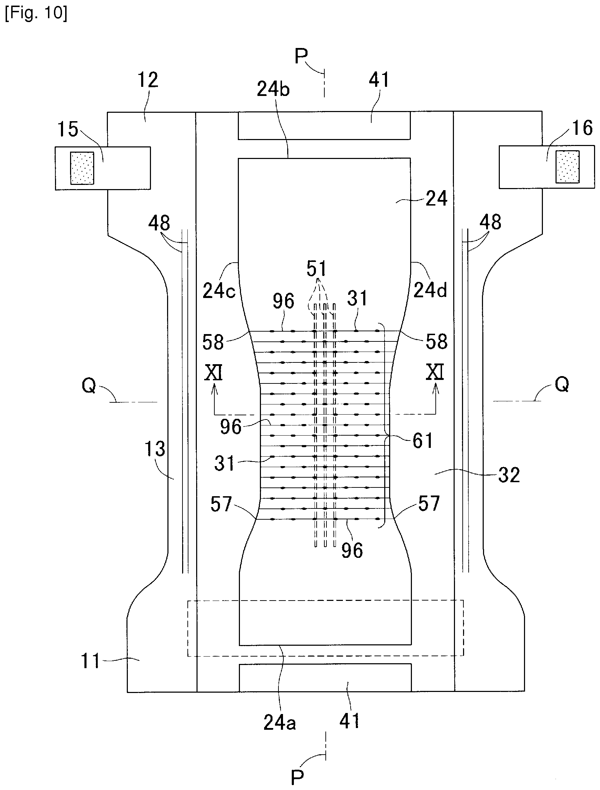

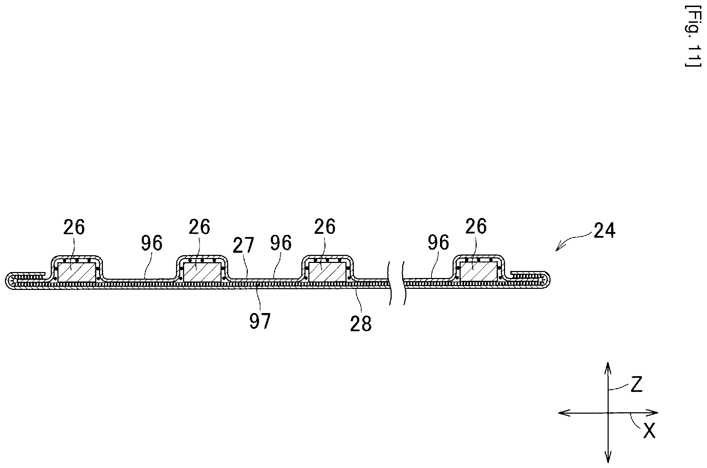

{Fig. 1} A perspective view of a disposable diaper as an example of the disposable diaper according to a first embodiment of the present invention.
{Fig. 2} A partially cutaway plan view of the diaper of Fig. 1 unfolded and as viewed from its skin-facing side.
{Fig. 3} A sectional view of the diaper of Fig. 1 taken along line III-III in Fig. 2.
{Fig. 4} A plan view of the unfolded diaper of Fig. 1 similar to Fig. 2, showing the diaper with an inner sheet and barrier sheets having been omitted.
{Fig. 5} An enlarged view of the region of the diaper of Fig. 1 enclosed by dashed-dotted line and denoted by V in the diaper of Fig. 4.
{Fig. 6} A sectional view of the diaper of Fig. 1 taken along line VI-VI in Fig. 4.
{Fig. 7} A diagram illustrating a bodily fluid absorbent structure in the diaper of Fig. 1 put on the wearer's body.
{Fig. 8} Diagram illustrating two phases (a) and (b) of a measuring method utilizing a doll.
{Fig. 9} A schematic diagram illustrating a part of a process to make the bodily fluid absorbent structure of the diaper of Fig. 1.
{Fig. 10} An unfolded plan view similar to Fig. 4, showing a disposable diaper according to a second embodiment of the present invention.
{Fig. 11} A sectional view of the diaper of Fig. 10 taken along line XI-XI in Fig. 10.
{Fig. 12} A diagram illustrating a bodily fluid absorbent structure in the diaper of prior art put on the wearer's body.

### {Description of Embodiments}

### <First Embodiment>

Details of a disposable diaper according to the present invention will be more fully understood from the description given hereunder with reference to the accompanying drawings. In Figs. 2 and 4, the longitudinal axis and the transverse axis of a diaper are denoted by P and Q, respectively.

It should be appreciated that a diaper 10 will be described hereunder in terms of a diaper for babies. Relating to Figs 1 to 3, the diaper 10 includes a chassis 14 having a longitudinal direction Y, a transverse direction X being orthogonal to the longitudinal direction Y, a skin-facing side, a non-skin-facing side, a front waist region 11, a rear waist region 12 and a crotch region 13 extending between the front and rear waist regions 11, 12; and a pair of tape-fastener tabs 15, 16 extending outward from opposite side edges 12b of the rear waist region 12 in the transverse direction X. The tape-fastener tabs 15, 16 are provided on the respective skin-facing side with first fastening zones 17, for example defined by hook elements of a so-called mechanical fastener. The front waist region 11 is provided on the non-skin-facing side with a second fastening zone 18 for example including a backing sheet made of a fibrous nonwoven fabric or a plastic film and loop elements of the mechanical fastener being set thereon.

In putting the diaper 10 on a wearer, the first fastening zones 17 on the respective tape-fastener tabs 15, 16 can be detachably engaged with the second fastening zone 18 to join each of the side edges of the front and rear waist regions 11, 12 and thereupon to define a waist-opening 20 and a pair of leg-openings 21 (See Fig. 1).

The chassis 14 includes a liquid-pervious inner sheet 22 lying on the skin-facing side, a liquid-impervious outer sheet 23 lying on the non-skin-facing side and a bodily fluid absorbent structure 24 interposed between the inner and outer sheets 22, 23. Both the inner and outer sheets 22, 23 may extend outward beyond a periphery of the bodily fluid absorbent structure 24 and may be bonded to each other outboard of the periphery of the bodily fluid absorbent structure 24 with hot melt adhesives, for example (not shown).

The inner sheet 22 may be formed of, for example, a nonwoven fabric made of hydrophilic fibers or hydrophobic fibers modified to become hydrophilic, a porous plastic film or a laminate sheet thereof. As the nonwoven fabric, for example, the following materials may be used: an air-through fibrous nonwoven fabric, a spun bonded fibrous nonwoven fabric or a point bonded fibrous nonwoven fabric having a basis mass in a range of about 20 to about 50 g/m², preferably in a range of about 20 to about 35 g/m², and a fiber density in a range of about 0.01 to about 0.04 g/cm³, preferably in a range of about 0.025 to about 0.035 g/cm³.

The outer sheet 23 defines the outer surface of the chassis 14 and may be formed of, for example, a moisture-pervious plastic film, a hydrophobic fibrous nonwoven fabric or a laminate sheet thereof. As the nonwoven fabric, for example, the following materials may be used: an air-through fibrous nonwoven fabric, a spun bonded fibrous nonwoven fabric or a point bonded fibrous nonwoven fabric having a basis mass in a range of about 15 to about 40 g/m², preferably in a range of about 25 to about 35 g/m², and a fiber density in a range of about 0.06 to about 0.10 g/cm³, preferably in a range of about 0.07 to about 0.09 g/cm³.

The bodily fluid absorbent structure 24 includes an absorbent core 26 formed of, for example, a mixture of absorbent fibers such as fluff wood pulp and super-absorbent polymer particles and at least one sheet for covering at least a major (central) part of a surface of the absorbent core 26; preferably an upper sheet 27 formed of for example a dispersant sheet such as a tissue paper or a liquid-pervious fibrous nonwoven fabric adapted to cover the upper surface of the absorbent core 26; and a lower sheet adapted to cover the lower surface of the absorbent core 26. The upper sheet 27 may be bonded to the absorbent core 26 with, for example, hot melt adhesives 29 applied in a curved line pattern such as in a wavy pattern or in an omega-pattern in which omega-like shaped curves are formed at intervals along one direction, and the lower sheet 28 may be fixed to the absorbent core 26 with, for example, hot melt adhesives 30 applied like a film (thin layer) . The lower sheet 28 may extend outward beyond the periphery of the absorbent core 26 and any such protruding segment may be fixed to the upper surface of the upper sheet 27 to cover the absorbent core 26 as a whole with the upper and lower sheets 27, 28. Though not shown in Fig. 3, the upper and lower sheets 27, 28 may be formed of a single sheet having a relatively wide width dimension.

The absorbent core 26 may be a mixture of absorbent fibers such as fluff wood pulp having a basis mass for example in a range of about 160 to about 180 g/m², super-absorbent polymer particles having a basis mass for example in a range of about 190 to about 210 g/m², and optionally thermoplastic synthetic fibers (staple fibers) having a basis mass for example in a range of about 10 to about 20 g/m², wherein a mass percentage of the super-absorbent polymer particles in the absorbent core 26 as a whole is preferably in a range of 40 to 60 %, so far as the effect of the present invention as will be described can be achieved.

As will be described in more detail, the bodily fluid absorbent structure 24, in the crotch region 13 and on the skin facing side, is provided with a plurality of debosses 31, in a zigzag-pattern (staggered pattern) as viewed in the longitudinal direction so that each of the adjacent debosses 31 may be spaced from each other in a given dimension in the longitudinal direction Y as well as in the transverse direction X. While the debosses 31 are formed on the side of the bodily fluid absorbent structure 24 facing the wearer's skin in one embodiment, the debosses 31 may alternatively be formed on the non-skin-facing side, or on both the skin-facing and non-skin-facing sides of the bodily fluid absorbent structure 24, so far as the intended effect of the present invention can be achieved.

Between the inner and outer sheets 22, 23, a leakage-barrier sheet 32 formed of a liquid-impervious, moisture-pervious plastic film may be fixed to the inner surface of one of the inner and outer sheets 22, 23, for example with hot melt adhesives (not shown) applied to the inner surface, to cover the non-skin-facing side of the bodily fluid absorbent structure 24. The leakage-barrier sheet 32 preferably has a sufficient length dimension extending outward beyond the entire peripheral edge of the bodily fluid absorbent structure 24 in order that bodily fluids seeping through the bodily fluid absorbent structure 24 can be prevented from leaking out. The diaper 10 may further include, on the skin-facing side, a pair of containment flaps 34, 35 provided outboard of the bodily fluid absorbent structure 24 as viewed in a transverse direction so as to be in a symmetric relationship about the longitudinal axis P.

The chassis 14 is contoured by front and rear ends 36, 37, opposed to each other in the longitudinal direction Y and extending in the transverse direction X, and side edges 38, 39, opposed to each other in the transverse direction X and extending in the longitudinal direction Y. The front and rear ends 36, 37, and the side edges 38, 39 are defined by respective segments of the inner sheet 22, the leakage-barrier sheet 32, the outer sheet 23 and the containment flaps 34, 35 extending outward beyond the front and rear ends 24a, 24b of the bodily fluid absorbent structure 24 in the longitudinal direction Y and extending beyond the side edges 24c, 24d of the bodily fluid absorbent structure 24 in the transverse direction X and overlapping one another. Between the inner sheet 22 defining the front and rear ends 36, 37, and the leakage-barrier sheet 32, waist elastics 41, for example made of an elastomer material such as natural rubber, synthetic rubber or urethane foam, may be interposed to extend in the transverse direction X.

In the front and rear waist regions 11, 12, the outer sheet 23 and the leakage-barrier sheet 32 extend outward beyond the side edges of the inner sheet 22, and the side edges of the leakage-barrier sheet 32 define the side edges 38, 39 in the transverse direction X. The segments of the outer sheet 23 and the leakage-barrier sheet 32 extending outward beyond the side edges 38, 39 respectively define front and rear side flaps 43, 44. Proximal ends 15a, 16a of the paired tape-fastener tabs 15, 16 are interposed between the outer sheet 23 and the containment flaps 34, 35 in the respective rear side flaps 44 and fixed to these elements 23, 34, 35 with hot melt adhesives (not shown) applied to the respective inner surfaces of these elements 23, 34, 35. Distal ends 15b, 16b of the tape-fastener tabs 15, 16 extend outward from the side edges of the rear side flaps 44 (i.e., the side edges 12b of the rear waist region 12) in the direction of the transverse axis Q as has previously been described. These distal ends 15b, 16b of the tape-fastener tabs 15, 16 are provided with the first fastener zones 17 on the skin-facing sides thereof.

The containment flaps 34, 35 respectively include proximal edges 45 defining portions of the side edges 38 and 39, front and rear fixed ends 46a, 46b fixed to the skin-facing side of the inner and outer sheets 22, 23 in the front and rear waist regions 11, 12 and distal edges 47 extending in the longitudinal direction Y between the front and rear fixed ends 46a, 46b and defined by folding inner side edges of the containment flaps 34, 35 inward. Outboard of the opposite side edges of the leakage-barrier sheet 32 as viewed in the transverse direction X, two strand-like or string-like leg elastics 48 extending in the longitudinal direction Y are attached under tension contractibly between the proximal edges 45 of the respective containment flaps 34, 35 and the opposite sided edges of the outer sheet 23. Two strand-like or string-like cuff elastics 49 extending in the longitudinal direction Y contractibly are attached in a contractible manner to each of the distal edges 47. With the diaper 10 put on the wearer's body, the distal edges 47 are spaced upward from the skin-facing side of the inner sheet 22 under contraction of the cuff elastics 49 and thereby can contain bodily waste inboard of the containment flaps 34, 35.

Referring to Figs. 2 and 3, the inner surface of the outer sheet 23 opposed to the skin-facing side of the bodily fluid absorbent structure 24 in the crotch region 13 may be provided with indicator means 51 including for example three line segments extending in the longitudinal direction Y. Each of the indicator means 51 may be formed of an ink layer adapted to change color and to be visually recognized from the outside when it gets wet with bodily waste such as urine. Though not shown, a cover sheet containing a sufficient quantity of surfactant agent may be interposed between the indicator means 51 and the outer sheet 23 and thereby to facilitate the indicator means 51 to be visually recognized.

Referring to Fig. 4, the opposite side edges 24c, 24d of the bodily fluid absorbent structure 24 are segmented, for convenience of illustration, into front side edges 53 rectilinearly extending across the front waist region 11 and then slightly curving inward in the crotch region 13, rear side edges 54 rectilinearly extending across the rear waist region 12 and then slightly curving inward in the crotch region 13, and concavely curved side edges 56 extending between the front and rear side edges 53, 54. The opposite side edges 24c, 24d respectively have front stiffness changing points 57 and rear stiffness changing points 58 corresponding to intersections of the front and rear side edges 53, 54 and the concavely curved side edges 56. A stiffness boundary line 61a connecting the front stiffness changing points 57, a stiffness boundary line 61b connecting the rear stiffness changingpoints 58, and the opposite concavely curved side edges 56, define a high stiffness region 61 in which a plurality of debosses are arranged, having a stiffness higher than the stiffness of the remaining region as a whole.

The present embodiment is exemplarily described herein on the basis of an S-sized diaper 10 for babies. Specifically, the length dimension L1 of the chassis 14 as measured in the longitudinal direction Y is in a range of about 380 to about 420mm and the length dimension L2 of the bodily fluid absorbent structure 24 as measured in the longitudinal direction Y is in a range of about 330 to about 360mm. More specifically, the length dimension L3 as measured from the front end 24a of the bodily fluid absorbent structure 24 to the stiffness boundary line 61a connecting the front stiffness changing points 57 is in a range of about 70 to about 90mm and the length dimension L4 as measured in the longitudinal direction Y from the stiffness boundary line 61a connecting the front stiffness changing points 57 to the stiffness boundary line 61b connecting the rear stiffness changingpoints 58 (i.e., a length dimension of the high stiffness region 61 as measured in the longitudinal direction Y) is in a range of about 130 to about 160mm. In addition, the distance L5 between the stiffness boundary line 61b connecting the rear stiffness changing points 58 and the rear end 24b of the bodily fluid absorbent structure as measured in the longitudinal direction Y is in a range of about 110 to about 130mm. As will be apparent from these specific dimensional values and the arrangement illustrated, the high stiffness region 61 is biased towards the front waist region 11.

Conventionally, when a bodily fluid absorbent structure 24 is formed with concavely curved side edges 56, such bodily fluid absorbent structure 24 tends to easily fit the wearer's thighs. However, a region of the absorbent core 26 between the opposite concavely curved side edges 56 may lose its initial shape due to being squeezed between the thighs. To overcome this problem, according to one embodiment, the high stiffness region 61 is defined between the opposite concavely curved side edges 56 and thereby the bodily fluid absorbent structure 24 can fit the wearer's body in the crotch region 13and in addition should not noticeably lose its initial shape; in consequence, the bodily fluid absorption performance thereof should not be deteriorated.

Referring to Figs. 5 and 6, the debosses 31 are arranged intermittently at regular intervals in a so-called zigzag-pattern (staggered pattern) in the longitudinal direction Y as well as in the transverse direction X. In Fig. 6, the thickness direction of the bodily fluid absorbent structure 24 is indicated by Z. The debosses 31 are formed by compressing the bodily fluid absorbent structure 24 from above using appropriate pressing means such as an debossing roller. Each deboss 31 has a density and a stiffness higher than that of a non-compressed zone. Specifically, the high stiffness region 61 includes transverse high stiffness zones 62 each defined by a plurality of debosses 31 arranged at regular intervals in transverse direction X and transverse low stiffness zones 63 each defined by a zone formed with none of the debosses 31. The high stiffness region 61 further includes longitudinal low stiffness zones 64 extending in the longitudinal direction Y each defined by a zone formed with none of the debosses 31. In Fig. 5, for convenience of illustration, the transverse low stiffness zone 63 is indicated by dots, the transverse high stiffness zone 62 is indicated by hatching, and the longitudinal low stiffness zone 64 is indicated by diagonal lines.

Each of the debosses 31 preferably has a generally elliptical shape which has a "height" in the longitudinal direction Y, in other words, a width dimension W1 of the respective transverse high stiffness zones 62, in a range of about 0.5 to about 3.0mm, preferably in a range of about 0.8 to about 1.5mm and a "width" in the transverse direction X in a range of about 2.0 to about 6.0mm, preferably about in a range of about 2.5 to about 4.0mm. The distance between each pair of debosses which are obliquely adjacent in the transverse direction X, in other words, a width dimension W3 of the longitudinal low stiffness zone 64, is in a range of about 3.0 to about 8.0mm, preferably in a range of about 4.0 to about 6.0mm, and a length dimension defined between each pair of the debosses 31 which are adjacent in the longitudinal direction Y, in other words, a width dimension W4 of the transverse low stiffness zone is in a range of about 4.0 to 10.0mm, preferably in a range of about 5.0 to about 8.0mm. A distance W5 between adjacent debosses 31 is in a range of about 5.0 to about 9.0mm and by setting the distance W5 at about 5.0mm or longer, the force exerted on the debosses 31 in the longitudinal direction Y can be dispersed, as will be described later in detail.

Referring to Fig. 6, a thickness dimension H1 of a non-compressed zone 66 defined between apair of adjacent debosses 31 is in a range of about 2.5 to about 3.5mm, preferably in a range of about 2.8 to about 3.2mm, and a thickness dimension H2 of each deboss 31 is in a range of about 1.0 to about 2.0mm, preferably in a range of about 1.5 to about 2.0mm. While the deboss 31 is illustrated by way of example as having a generally elliptical shape which is relatively long in the transverse direction X, the shape of the deboss 31 may be selected from other various shapes such as rectangle, rhombus and isosceles triangle, so far as the effect of the present invention can be achieved, in other words, the length dimension W2 (width) in the transverse direction X is larger than the length dimension W1 (height) in the longitudinal direction Y.

Referring again to Fig. 4, the indicator means 51 are preferably formed between each pair of the adjacent longitudinal low stiffness zones 64 to extend in the longitudinal direction Y and to overlap the plural arrays of the debosses 31 each arranged intermittently in the longitudinal direction Y. The indicator means 51 are formed in this manner to overlap zones in which the debosses 31 are intermittently arranged. These zones are strongly resistant to getting creased in the longitudinal direction Y and, in consequence, the visibility of the indicator means 51 should not be hindered by longitudinal creases.

Referring to Fig. 7 showing the diaper 10 according to the present embodiment as put on the wearer's body, a height dimension H3 of ridge 70 facing the wearer' s crotch region (i.e., a dimension as measured from respective lower ends of the opposite concavely curved side edges 56 to an apex 70a of the ridge 70) is smaller than that in the diaper 110 of the prior art, as will be apparent from a comparison with Fig. 12 which illustrates the bodily fluid absorbent structure 124 in the diaper of the prior art as put on the wearer's body. This is for the reason described below. With the diaper 110 of prior art put on the wearer's body, the bodily fluid absorbent structure 24 lying in the middle of the crotch region 13 may be formed with relatively significant longitudinal creases 171 as the structure 24 is squeezed between the wearer's thighs and may be forced to be displaced inward such that the ridge 170 might be formed. Such a ridge 170 may dig into the wearer's bottom cleavage and not only make the wearer uncomfortable but also cause bodily fluids to move along the ridge and eventually leak sideways beyond the leg-openings.

In the diaper 10 according to the present embodiment, the bodily fluid absorbent structure 24 is formed with the high stiffness region 61 including a plurality of debosses 31, and consequently the stiffness in this region 61 is higher than in the remaining zone. In this way, the bodily fluid absorbent structure 24 is rarely twisted in this zone and is strongly resistant to the development of longitudinal creases. Particularly for the reason that each of the debosses 31 is shaped to be relatively long in the transverse direction X, sufficiently high stiffness in the transverse direction X is assured in the high stiffness region 61 to prevent the absorbent core 26 from significantly losing its initial shape even when the high stiffness region 61 is squeezed in the wearer's crotch region. With the diaper 10 put on the wearer's body, plural transverse creases 72 extending in the transverse direction X appear in the transverse low stiffness zones 63 and function to alleviate the development of any deep longitudinal creases. In consequence, the ridge 70 does not protrude upward to an unacceptable extent. Also in the longitudinal direction Y, the low stiffness zones 64 are formed and the longitudinal creases 71 also appear in these zones 64. However, as will be apparent from Fig. 5, the width dimension W4 of the transverse low stiffness zone 63 is larger than the width dimension W3 of the longitudinal low stiffness zone 64 and the range in which the transverse creases 72 are formed is wider than the range in which the longitudinal creases 71 are formed. Consequently, development of the longitudinal creases 71 can be restricted or, at least, development of relatively large and deep longitudinal creases 71 can be restricted.

While the arrangement of the debosses 31 in a zigzag-pattern inevitably leads to the development of relatively small transverse creases 72 in the transverse high stiffness zones between each pair of adjacent debosses 31, such transverse creases 72 are finely dispersed and therefore these small transverse creases do not link with one another and do not cooperate with the transverse low stiffness zone 63 to form relatively significant transverse creases 72. Referring to Fig. 5, a force F1 in the longitudinal direction Y which would lead to development of the longitudinal creases 71 is exerted on the respective debosses 31 when the crotch region 13 is squeezed between the wearer's thighs. However, the deboss 31 has a shape which is relatively long in the transverse direction X and, as indicated by arrows F2, F3, such force is dispersed toward the obliquely adjacent debosses 31. Consequently, development of the longitudinal creases 71 is restricted and it is correspondingly difficult to maintain a temporary shape and height of the ridge 70.

In this way, comparing to a diaper of the prior art, the ridge 70 of the bodily fluid absorbent structure 24 is relatively low and the shape of the ridge 70 is not maintained. In consequence, the ridge 70 should not dig into the wearer's bottom cleavage 81. Specifically, to achieve the effect of the present invention, the height dimension H3 of the convexly ridge 70 is preferably about 33mm or less as indicated by the measurement result of the "compression test utilizing a doll" as described hereunder.

In order to define the high stiffness region 61 it may be possible to use, for example, flat press working to uniformly compress an area until the area has a thickness the same as that of the debosses 31, and thereby increase the stiffness thereof so that development of the longitudinal creases can be restricted. In this case, however, the high stiffness region 61 as a whole will have uniformly high stiffness, and the absorbent core 26 may be deteriorated in flexibility as well as in cushioning properties and eventually may be uncomfortable to wear, and the absorption capacity of the core 26 may also be deteriorated. In contrast, in the diaper 10 according to the present embodiment, the debosses 31 are arranged in a zigzag-pattern so that the transverse high stiffness zones 62 and the transverse low stiffness zones 63 extending in the transverse direction X are alternately defined in the longitudinal direction Y in the high stiffness region 61. With such a unique arrangement, the stiffness of the high stiffness region 61 as a whole does not be unacceptably increased. Therefore, the diaper 10 does not create an uncomfortable feeling against the wearer even when the high stiffness region 61 is squeezed between the wearer's thighs.

The crotch region 13 is apt to come into tight contact with the inguinal region of the wearer under a rather intense compression in the transverse direction X. Considering this, the high stiffness region 61 is formed at the section of the crotch region 13 biased toward the front waist region 11 so that development of the longitudinal creases 71 due to such compression may be restricted.

Stiffness values in the transverse direction X in the section of the bodily fluid absorbent structure 24 formed with the high stiffness region 61 and in the section of the bodily fluid absorbent structure not formed with the high stiffness region were measured by Taber's method in accordance with JIS P 8125. A series of measurements indicated that the former is in a range of 0.027 to 0.049N/m and the latter is in a range of 0.012 to 0.033N/m. The measurement was conducted for the respective zones on the basis of test pieces each having the longitudinal dimension of 38mm x the transverse dimension of 70mm and, as the measuring device, a Taber Stiffness Tester manufactured by Yasuda Seiki Seisakusho LTD. was used.

### <Compression Test using a doll>

Figs. 8 (a) and 8(b) illustrate how to conduct the compression test using doll. The measurement result of the compression test for the bodily fluid absorbent structure 24 using a doll simulating the diaper wearer is indicated in Table 1. A summary of the compression test is given below. The disposable diaper according to the present invention including the bodily fluid absorbent structure 24 formed with the high stiffness region 61 was used as the inventive example. A disposable diaper including the bodily fluid absorbent structure not formed with the high stiffness region 61 and therefore having a uniform stiffness was used as a comparative example 1. A disposable diaper including the bodily fluid absorbent structure formed with the high stiffness region 61 including the same debosses 31 as those according to the present invention in size as well as in shape but relatively long in the longitudinal direction Y was used as a comparative example 2. A disposable diaper including the bodily fluid absorbent structure flat press-worked to have a substantially uniform stiffness (0.035N/m approximately equivalent to the stiffness value in the high stiffness region 61) was used as a comparative example 3. It should be noted here that, in the respective disposable diapers used for the compression test, the stiffness value in the high stiffness region of the bodily fluid absorbent structure was 0.036N/m and the stiffness value in the remaining zone of the bodily fluid absorbent structure was 0.025N/m.

**{Table 1}**

| | Width dimension (mm) of the bodily fluids absorbent structure in the midsection of the crotch region | Height dimension (nnn) of the ridge in the midsection of the crotch region |
|---|---|---|
| Inventive Example | 45 | 30 |
| Comparative Example 1 | 35 | 35 |
| Comparative Example 2 | 35 | 35 |
| Comparative Example 3 | 40 | 37 |

### <Measuring Method>

For every measurement on the inventive example, the comparative example 1, the comparative example 2 and the comparative example 3, the respective diapers were put on the doll made of silicon and a transverse center line was drawn along the transverse axis of the respective diapers with ink. Then the transverse center line of the respective diaper was aligned with a longitudinal central part of the crotch region of the doll to adjust so that the respective outer ends of the front waist region and the rear waist region of the diaper may come at a generally same level. The doll was prepared so that an S-size diaper fits the doll well. Specifically, a width dimension of the inguinal region is about 240mm, a circumferential dimension of respective leg-openings is about 265mm, a circumferential waist dimension is about 390mm, a circumferential buttock dimension is about 380mm and a width dimension (i.e., transverse length dimension) of central portion in the crotch region is about 25mm.

Referring to Fig. 8(a), a series of actions, specifically, closing the doll's opened legs and opening the doll's closed legs are repeated ten (10) times at a rate of 100 to 140° per second under the above-mentioned dimensional conditions. In this measurement, the angle between two straight lines each extending from the toe to the groin is denoted as the angle alfa and this angle alfa is 75° in the state of the legs having been fully opened and 0° in the state of the legs having been fully closed. Now the action of closing the doll's opened legs and opening the doll's closed legs is repeated ten (10) times.

Referring now to Fig. 8 (b), the doll is placed on a measuring table with the legs fully closed to lie face up, and from this state the doll' s upper limbs are vertically raised ten (10) times. Then, the doll is placed on the measuring table with the legs fully opened and the diaper is taken off from the doll with a deformation of the bodily fluid absorbent structure in the crotch region of the diaper due to the above-mentioned series of actions maintained.

In the middle of the inked up line in the respective diapers, having been taken off from the doll, the height of the region of the bodily fluid absorbent structure convexly raised toward the bottom cleavage of the doll, specifically, the distance from the apex of the above-mentioned region to the lower end thereof was denoted as "height dimension (mm) of the ridge in the midsection of the crotch region", and the width dimension (i.e., transverse length dimension) of the midsection of the inked up line was designated as "width dimension (mm) of the bodily fluid absorbent structure in the midsection of the crotch region".

### <Measurement Result>

As will be apparent from Table 1, the height dimension of the ridge in the midsection of the crotch region is smaller in the inventive example than in the comparative examples 1 through 3 and the width dimension of the bodily fluid absorbent structure in the midsection of the crotch region is larger in the inventive example than in the comparative examples 1 through 3. This is for the reason that the bodily fluid absorbent structure in the diaper as the inventive example is formed with much more transverse creasing, servingto restrict development of longitudinal creases, than those in the diapers of the comparative examples. In consequence, the ridge is lower in the inventive example than in the comparative examples and the width dimension of the bodily fluid absorbent structure is correspondingly larger in the inventive example than in the comparative examples. In this manner, it is possible for the inventive example to avoid more reliably the problem that the ridge of the bodily fluid absorbent structure might dig into the bottom cleavage of the doll (wearer).

Fig. 9 is a schematic diagram illustrating a part of a process to make the bodily fluid absorbent structure. In Fig. 9, a machine direction is designated by MD. A region in Fig. 9 enclosed by dashed-dotted line is a part of a roller 93 used in a second pressing step illustrated in an enlarged scale.

Referring to Fig. 9, a first web 85 of original fabric for the lower sheet 28 is conveyed by a conveyor belt 84 and the absorbent cores 26 are successively transferred from a rotary drum 86 onto the first web 85. Then, a second web 87 is transported from the upstream and lain over the absorbent cores 26 from above. The inner surface of the first web 85 is coated, in a first coating step 88, with hot melt adhesives applied like a film and the inner surface of the second web 87 is coated, in a second coating step 89, with hot melt adhesives in an omega-pattern. Now in a first press working step using a roller 90 having a smooth outer peripheral surface and an anvil roll 91 opposed to the roller 90, the first web 85 and the second web 87 with the absorbent cores 26 interposed therebetween are subjected to flat press working so that the assembly of these first and second webs 85, 87 are bonded together with the absorbent cores 26 with hot melt adhesives applied to the respective inner surfaces of the first and second webs 85, 87 to form a laminate web 92. Then, in a second press working step using roller 93 having a plurality of bosses 93a planted on a part of its outer peripheral surface and an anvil roll 94 opposed to the roller 93, a predetermined region of the laminate web 92 is debossed by pressing a plurality of bosses 93a formed on the outer peripheral surface of the roller against the predetermined region to form the high stiffness zone 62. After this debossing, the laminate web 92 is cut in the cutting step (not shown) to obtain the individual bodily fluid absorbent structures 24. Each of the bosses 93a of the roller 93 has a generally elliptical shape which is relatively long in the direction extending orthogonally to the machine direction MD (i.e., the width direction of the conveyor belt 84).

As will be obviously understood from the above description, the bodily fluid absorbent structure 24 as a whole has been subjected to the flat press working before it is debossed. Consequently, the not debossed portion of the bodily fluid absorbent structure 24 and the non-compressed zone 66 of the high stiffness region 61 have a uniform thickness (about in a range of 2.0 to 2.5mm), on one hand, and the shape of the absorbent core 26 is stabilized by the flat press working, on the other hand. In this way, the debossing is relatively easy.

As will be understood from the part of the peripheral surface of the roller 93 enclosed by dashed-dotted line and shown in an enlarged scale in Fig. 9, a plurality of the bosses 93a formed on a part of the outer peripheral surface of the roller 93 respectively preferably have a length dimension L6 of about 3.0mm. The bodily fluid absorbent structure 24 preferably has a thickness dimension in a range of 2.0 to 2.5mm after the structure 24 has been flat press worked, and each of the bosses 93a of the roller 93 preferably has the length dimension L6 of about 3.0mm, so that spots on the bodily fluid absorbent structure 24 coming in contact with the bosses 93a are compressed but the remaining zone maintains the thickness immediately after being flat press worked. A differential thickness dimension of the debosses 31 and the remaining zone may be regulated so that the thickness dimension difference between the debosses 31 and the remaining zone preferably may be kept to about 0.5mm or less. In this way, the contact surface of the bodily fluid absorbent structure 24 as a whole can be kept generally uniform and thereby the wearer' s feeling to wear can be prevented from being deteriorated due to irregularity of the inner surface of the bodily fluid absorbent structure 24.

An inclination angle alfa included between the outer peripheral surface of the roller 93 and the boss 93a is preferably about 130°. The boss 93a preferably has a generally elliptical shape which is relatively long in a direction orthogonal to the machine direction MD. Although it will be not easy for the bosses 93a to be separated from the laminate web 90 under ordinary circumstances, the inclination angle of the bosses 93a to the outer peripheral surface of the roller 93 may be set to be gentle according to the present embodiment to prevent the bosses 93a from being caught by the surface of the laminate web 90 and from damaging some part of the laminate web 90.

After producing the diaper 10, a thickness meter (Digital Thickness JA-257 manufactured by PEACOCK Corporation) was used to measure the thickness of the zone of the bodily fluid absorbent structure 24 in the crotch region 13 of the diaper 10 in which the high stiffness region 61 is formed and the zone in which the high stiffness region 61 is not formed after the bodily fluid absorbent structure 24 has not been creased in the longitudinal direction as well as in the transverse direction. The measurement result indicated that a thickness difference between the region in which the high stiffness region 61 is formed and the region in which the high stiffness region 61 is not formed at all is 0.5mm or less. In view of such measurement result, the crotch region 13 of the diaper 10 as a whole can be determined to have a smooth surface.

### <Second Embodiment>

Fig. 10 is a view similar to Fig. 4, showing a second embodiment of the diaper according to the present invention and Fig. 11 is a view similar to Fig. 6, showing the second embodiment of the diaper 10 according to the present invention. The basic construction of the diaper 10 according to this embodiment is substantially the same as that of the first embodiment and only the feature different from that of the first embodiment will be described hereunder.

In the diaper 10 according to the present embodiment, a groove 96 extending in the transverse direction X is formed between adjacent debosses 31 in the bodily fluid absorbent structure 24. In each of the grooves 96, the absorbent core 26 is substantially not present so that the inner surface of the upper sheet 27 and the inner surface of the lower sheet 28 overlap and are bonded to each other by the intermediary of a joining layer formed of hot melt adhesives 29, 30. According to the present embodiment, the grooves 96 may be formed to keep the stiffness of the high stiffness region 61 as a whole moderate. In this way, it is possible to restrict an excessive increase of stiffness in the high stiffness region 61 due to the presence of the debosses 31.

The component members of the diaper 1 are not limited to those described in this specification but other various types of material widely used in the relevant technical field may be used without limitation. Terms "first" and "second" used in the specification and claims of the present invention are used merely to distinguish the similar elements, similar positions or other similar means.

The first aspects described above may be arranged in at least the following item:
(i) A disposable diaper including a chassis having a longitudinal direction, a transverse direction, a skin-facing side, a non-skin-facing side, a front waist region, a rear waist region, a crotch region extending in the longitudinal direction between the front and rear waist regions, and a bodily fluid absorbent structure extending in the longitudinal direction across the crotch region into the front and rear waist regions, wherein a region of the bodily fluid absorbent structure extending at least in a central area thereof is formed with a high stiffness region including a plurality of debosses spaced one from another in predetermined dimensions in each of the longitudinal direction and the transverse direction, wherein:
   the bodily fluid absorbent structure includes an absorbent core and an upper sheet covering a surface of the absorbent core;
   the debosses are shaped so that the length dimension thereof in the transverse direction (width) is longer than the length dimension thereof in the longitudinal direction (height);
   the high stiffness region includes a plurality of longitudinal low stiffness zones extending in the longitudinal direction and formed with none of the debosses, a plurality of transverse high stiffness zones formed with the debosses arranged at regular intervals in the transverse direction, and transverse low stiffness zones lying between each pair of the transverse high stiffness zones and formed with none of the debosses, the transverse low stiffness zones and transverse high stiffness zones being adjacent in the longitudinal direction;
   in the high stiffness region, the width dimension of the transverse low stiffness zones in the longitudinal direction is larger than the width dimension of the longitudinal low stiffness zones in the transverse direction; and
   the high stiffness region is formed in an area of the crotch region biased toward the front waist region.

One or more aspect described in the above item (i) may provide one or more of the following advantageous effects:
(a) The debosses forming the high stiffness region of the bodily fluid absorbent structure are respectively shaped so that a length dimension thereof in the transverse direction (width) is longer than a length dimension thereof in the longitudinal direction (height) ; the high stiffness region includes the transverse high stiffness zones formed of the debosses intermittently arranged in the transverse direction; and the transverse low stiffness zones each defined between a pair of adjacent transverse high stiffness zones. With this unique arrangement, a plurality of the transverse creases can be easily developed on the bodily fluid absorbent structure of the diaper put on the wearer's body. Development of the transverse creases advantageously restricts development of the longitudinal creases in the longitudinal low stiffness zones which may cause the central portion of the bodily fluid absorbent structure to be convexly raised up toward the wearer's crotch region. In consequence, such ridge can be restricted as low as possible and such ridge should not dig into the wearer's bottom cleavage.

The debosses are arranged in a zigzag-pattern on the skin-facing side or on the non-skin-facing side, or on both the skin-facing and non-skin-facing sides.

Additionally, one or more of the following non-limiting embodiments are provided in accordance with further aspects:
(i) Each pair of adjacent debosses are spaced from each other by about 5.0mm or more.
(ii) Each pair of adjacent debosses are spaced from each other by about 5.0mm to about 9.0mm
(iii) The high stiffness region is formed, between each pair of the debosses being adjacent in the transverse direction, with grooves extending in the transverse direction and substantially not occupied by the absorbent core.
(iv) The absorbent core has side edges concavely curved inward in the central zone of the crotch region, and the high stiffness region is formed between the concavely curved side edges.
(v) In the crotch region, a differential thickness dimension between the area of the bodily fluid absorbent structure formed with the high stiffness region and the area of the bodily fluid absorbent structure not formed with the high stiffness region is about 0.5mm or less.

According to the embodiments in the above (i) to (v), the advantageous effect (s) set forth at (a) is/are better ensured. Further advantageous effects of the respective embodiments may be obtained as discussed in the respective related descriptions.

## Claims

1. A disposable diaper (10) having a longitudinal direction (Y) and a transverse direction (X), comprising:
a chassis (14) including a skin-facing side, a non-skin-facing side, a front waist region (11), a rear waist region (12) and a crotch region (13) extending in the longitudinal direction between the front and rear waist regions; and
a bodily fluid absorbent structure which comprises an absorbent core (26) and at least one sheet covering a surface of the absorbent core;
the bodily fluid absorbent structure (24) extends in the longitudinal direction across the crotch region into the front and rear waist regions, wherein a region of the bodily fluid absorbent structure extending at least in a central area thereof is formed with a high stiffness region (61) comprising a plurality of debosses (31) spaced one from another in predetermined dimensions in the longitudinal and transverse directions, **characterised in that**:
the debosses (31) are respectively shaped so that the length dimension thereof in the transverse direction is longer than the length dimension thereof in the longitudinal direction;
the high stiffness region is formed in an area of the crotch region biased toward the front waist region;
the high stiffness region includes a plurality of longitudinal low stiffness zones (64) extending in the longitudinal direction and formed with none of the debosses, a plurality of transverse high stiffness zones (62) formed with the debosses arranged at regular intervals in the transverse direction, and transverse low stiffness zones (63) each lying between each pair of the transverse high stiffness zones and formed with none of the debosses;
the transverse low stiffness zones and transverse high stiffness zones are adjacent in the longitudinal direction;
in the high stiffness region, the width dimension (W4) of the transverse low stiffness zones (63) in the longitudinal direction is larger than the width dimension (W3) of the longitudinal low stiffness zones (64) in the transverse direction; and
the debosses are arranged in a zigzag-pattern on the skin-facing side or on the non-skin-facing side, or on both the skin-facing and non-skin-facing sides of the bodily fluid absorbent structure.

2. The diaper defined by claim 1, wherein adjacent debosses are spaced from each other by about 5.0mm to about 9.0mm.

3. The diaper defined by any preceding claim, wherein grooves (96) extending in the transverse direction are formed, between each pair of the debosses being adjacent in the transverse direction, and wherein, in each of the grooves, the absorbent core (26) is not present.

4. The diaper defined by any preceding claim, wherein the absorbent core has side edges (56) concavely curved inward in a central zone of the crotch region and the high stiffness region (61) is formed between the concavely curved side edges.

5. The diaper defined by any preceding claim, wherein in the crotch region, a differential thickness dimension between the area of the bodily fluid absorbent structure formed with the high stiffness region and the area of the bodily fluid absorbent structure not formed with the high stiffness region is about 0.5mm or less.

6. The diaper defined by any preceding claim, wherein the chassis (14) includes a liquid-pervious inner sheet (22) lying on the skin-facing side, a liquid-impervious outer sheet (23) lying on the non-skin-facing side and the bodily fluid absorbent structure (24) is interposed between the inner and outer sheets;
the inner surface of the outer sheet (23)opposed to the skin-facing side of the bodily fluid absorbent structure in the crotch region (13) is provided with indicator means (51);
the indicator means is formed of an ink layer adapted to change color and to be visually recognized from the outside when the ink layer gets wet with bodily waste; and
the indicator means are formed between each pair of the adjacent longitudinal low stiffness zones (64) to extend in the longitudinal direction and to overlap a plurality of arrays of the debosses (31) each arranged intermittently in the longitudinal direction.

## Patentansprüche

1. Einwegwindel (10), die eine Längsrichtung (Y) und eine Querrichtung (X) aufweist, die Folgendes umfasst:
einen Grundkörper (14), der Folgendes einschließt: eine der Haut zugewandte Seite, eine nicht der Haut zugewandte Seite, einen vorderen Taillenbereich (11), einen hinteren Taillenbereich (12) und einen Schrittbereich (13), der sich in der Längsrichtung zwischen dem vorderen und hinteren Taillenbereich erstreckt; und
eine Körperflüssigkeit absorbierende Struktur, die einen Saugkern (26) und mindestens eine Lage, die eine Oberfläche des Saukerns bedeckt, umfasst;
wobei sich die Körperflüssigkeit absorbierende Struktur (24) in der Längsrichtung über den Schrittbereich in den vorderen und hinteren Taillenbereich erstreckt, wobei ein Bereich der Körperflüssigkeit absorbierenden Struktur, die sich mindestens in einer zentralen Fläche davon erstreckt, mit einem Bereich mit hoher Steifigkeit (61) ausgebildet ist, der eine Vielzahl von Einbuchtungen (31) umfasst, die in vorgegebenen Abmessungen in der Längs- und Querrichtung voneinander beabstandet sind, **dadurch gekennzeichnet, dass**:
die Einbuchtungen (31) jeweils derart geformt sind, dass die Längenabmessung davon in der Querrichtung länger ist als die Längenabmessung davon in der Längsrichtung;
der Bereich mit hoher Steifigkeit in einer Fläche des Schrittbereichs, in Richtung des vorderen Taillenbereichs ausgerichtet, ausgebildet ist;
der Bereich mit hoher Steifigkeit Folgendes einschließt: eine Vielzahl von längs verlaufenden Zonen mit geringer Steifigkeit (64), die sich in der Längsrichtung erstrecken und ohne die Einbuchtungen ausgebildet sind, eine Vielzahl von quer verlaufenden Zonen mit hoher Steifigkeit (62), die mit den Einbuchtungen ausgebildet sind, die in regelmäßigen Abständen in der Querrichtung angeordnet sind, und quer verlaufende Zonen mit geringer Steifigkeit (63), die jeweils zwischen jedem Paar der quer verlaufenden Zonen mit hoher Steifigkeit liegen und ohne die Einbuchtungen ausgebildet sind;
die quer verlaufenden Zonen mit geringer Steifigkeit und quer verlaufenden Zonen mit hoher Steifigkeit in der Längsrichtung benachbart sind;
in dem Bereich mit hoher Steifigkeit die Breitenabmessung (W4) der quer verlaufenden Zonen mit geringer Steifigkeit (63) in der Längsrichtung größer ist als die Breitenabmessung (W3) der längs verlaufenden Zonen mit geringer Steifigkeit (64) in der Querrichtung; und
die Einbuchtungen in einem Zickzackmuster auf der der Haut zugewandten Seite oder auf der nicht der Haut zugewandten Seite oder auf sowohl der der Haut zugewandten als auch der nicht der Haut zugewandten Seite der Körperflüssigkeit absorbierenden Struktur angeordnet sind.

2. Windel wie durch Anspruch 1 definiert, wobei benachbarte Einbuchtungen durch etwa 5,0 mm bis etwa 9,0 mm voneinander beabstandet sind.

3. Windel wie durch einen vorstehenden Anspruch definiert, wobei Rillen (96), die sich in der Querrichtung erstrecken, zwischen jedem Paar der Einbuchtungen, die in der Querrichtung benachbart sind, ausgebildet sind und wobei in jeder der Rillen der Saugkern (26) nicht vorliegt.

4. Windel wie durch einen vorstehenden Anspruch definiert, wobei der Saugkern Seitenkanten (56) aufweist, die nach innen in einer zentralen Zone des Schrittbereichs konkav gekrümmt sind, und der Bereich mit hoher Steifigkeit (61) zwischen den konkav gekrümmten Seitenkanten ausgebildet ist.

5. Windel wie durch einen vorstehenden Anspruch definiert, wobei in dem Schrittbereich eine differenzielle Dickenabmessung zwischen der Fläche der Körperflüssigkeit absorbierenden Struktur, die mit dem Bereich mit hoher Steifigkeit ausgebildet ist, und der Fläche der Körperflüssigkeit absorbierenden Struktur, die nicht mit dem Bereich mit hoher Steifigkeit ausgebildet ist, etwa 0,5 mm oder weniger beträgt.

6. Windel wie durch einen vorstehenden Anspruch definiert, wobei der Grundkörper (14) Folgendes einschließt: eine flüssigkeitsdurchlässige innere Lage (22), die auf der der Haut zugewandten Seite liegt, eine flüssigkeitsundurchlässige äußere Lage (23), die auf der nicht der Haut zugewandten Seite liegt, und die Körperflüssigkeit absorbierende Struktur (24) zwischen die innere und äußere Lage eingefügt ist;
die innere Oberfläche der äußeren Lage (23), die der der Haut zugewandten Seite der Körperflüssigkeit absorbierenden Struktur in dem Schrittbereich (13) gegenüberliegt, mit Indikatormittel (51) bereitgestellt ist;
das Indikatormittel aus einer Farbschicht gebildet ist, die zur Farbänderung und zur visuellen Erkennung von außen, wenn die Farbschicht durch Körperausscheidung feucht wird, geeignet ist; und
die Indikatormittel zwischen jedem Paar der benachbarten, längs verlaufenden Zonen mit geringer Steifigkeit (64) ausgebildet sind, um sich in der Längsrichtung zu erstrecken und mit einer Vielzahl von Anordnungen der Einbuchtungen (31), die jeweils in Abständen in der Längsrichtung angeordnet sind, zu überlappen.

## Revendications

1. Couche jetable (10) ayant une direction allant dans le sens longitudinal (Y) et une direction allant dans le sens transversal (X), comportant :
une armature (14) comprenant un côté orienté vers la peau, un côté non orienté vers la peau, une région avant au niveau de la taille (11), une région arrière au niveau de la taille (12) et une région au niveau de l'entrejambe (13) s'étendant dans la direction allant dans le sens longitudinal entre les régions avant et arrière au niveau de la taille ; et
une structure absorbant des fluides corporels qui comporte une partie centrale absorbante (26) et au moins une feuille recouvrant une surface de la partie centrale absorbante ;
la structure absorbant des fluides corporels (24) s'étend dans la direction allant dans le sens longitudinal en travers de la région au niveau de l'entrejambe jusque dans les régions avant et arrière au niveau de la taille, dans laquelle une région de la structure absorbant des fluides corporels s'étendant au moins dans une partie centrale de celle-ci est formée avec une région de haute rigidité (61) comportant une pluralité de creux (31) espacés les uns par rapport aux autres en des dimensions prédéterminées dans les directions allant dans le sens longitudinal et le sens transversal,
**caractérisée en ce que** :
les creux (31) sont formés respectivement de telle sorte que la dimension de longueur de ceux-ci dans la direction allant dans le sens transversal est plus longue que la dimension de longueur de ceux-ci dans la direction allant dans le sens longitudinal ;
la région de haute rigidité est formée dans une zone de la région au niveau de l'entrejambe sollicitée vers la région avant au niveau de la taille ;
la région de haute rigidité comprend une pluralité de zones de faible rigidité longitudinales (64) s'étendant dans la direction allant dans le sens longitudinal et formées avec aucun des creux, une pluralité de zones de haute rigidité transversales (62) formées avec les creux agencés selon des intervalles réguliers dans la direction allant dans le sens transversal, et des zones de faible rigidité transversales (63) reposant chacune entre chaque paire des zones de haute rigidité transversales et formées avec aucun des creux ;
les zones de faible rigidité transversales et les zones de haute rigidité transversales sont adjacentes dans la direction allant dans le sens longitudinal ;
dans la région de haute rigidité, la dimension de largeur (W4) des zones de faible rigidité transversales (63) dans la direction allant dans le sens longitudinal est supérieure à la dimension de largeur (W3) des zones de faible rigidité longitudinales (64) dans la direction allant dans le sens transversal ; et
les creux sont agencés selon une configuration en zigzag sur le côté orienté vers la peau ou sur le côté non orienté vers la peau, ou sur les deux côtés orienté vers la peau et non orienté vers la peau de la structure absorbant des fluides corporels.

2. Couche selon la revendication 1, dans laquelle des creux adjacents sont espacés les uns par rapport aux autres selon une distance allant d'environ 5,0 mm à environ 9,0 mm.

3. Couche selon l'une quelconque des revendications précédentes, dans laquelle des rainures (96) s'étendant dans la direction allant dans le sens transversal sont formées, entre chaque paire des creux qui sont adjacents dans la direction allant dans le sens transversal, et dans laquelle, dans chacune des rainures, la partie centrale absorbante (26) n'est pas présente.

4. Couche selon l'une quelconque des revendications précédentes, dans laquelle la partie centrale absorbante a des bords latéraux (56) courbes de manière concave vers l'intérieur dans une zone centrale de la région au niveau de l'entrejambe et la région de haute rigidité (61) est formée entre les bords latéraux courbes de manière concave.

5. Couche selon l'une quelconque des revendications précédentes, dans laquelle, dans la région au niveau de l'entrejambe, une dimension d'épaisseur différentielle entre la zone de la structure absorbant des fluides corporels formée avec la région de haute rigidité et la zone de la structure absorbant des fluides corporels non formée avec la région de haute rigidité est d'environ 0,5 mm ou moins.

6. Couche selon l'une quelconque des revendications précédentes, dans laquelle l'armature (14) comprend une feuille intérieure perméable aux liquides (22) reposant sur le côté orienté vers la peau, une feuille extérieure imperméable aux liquides (23) reposant sur le côté non orienté vers la peau et la structure absorbant des fluides corporels (24) est intercalée entre les feuilles intérieure et extérieure ;
la surface intérieure de la feuille extérieure (23) opposée au côté orienté vers la peau de la structure absorbant des fluides corporels dans la région au niveau de l'entrejambe (13) comporte des moyens indicateurs (51) ;
le moyen indicateur est formé à partir d'une couche d'encre adaptée pour changer de couleur et pour être visuellement reconnue de l'extérieur quand la couche d'encre s'humidifie sous l'effet de déchets corporels ; et
les moyens indicateurs sont formés entre chaque paire de zones de faible rigidité longitudinales adjacentes (64) pour s'étendre dans la direction allant dans le sens longitudinal et pour chevaucher une pluralité de séries de creux (31) agencés chacun de manière intermittente dans la direction allant dans le sens longitudinal.
